# EUROPEAN PATENT SPECIFICATION

(11) **EP 3 620 117 B1**
(45) Date of publication and mention of the grant of the patent: **12.01.2022**
(21) Application number: 19192492.7
(22) Date of filing: 20.08.2019
(51) Int. Cl.: A61B 17/08, A61B 17/10, A61B 17/122, A61B 17/128

(54) **LARGE WOUND AREA ANASTOMOSIS CLIP**
EIN ANASTOMOSECLIP FÜR GROSSFLÄCHIGE WUNDE
CLIP D'ANASTOMOSE POUR PLAIE DE GRANDE TAILLE

(30) Priority: 06.09.2018 CN 201811037943
(43) Date of publication of application: 11.03.2020
(73) Proprietor: Zhejiang Pengtian Medical Instrument Co., Ltd., Zhuji, Zhejiang Province 311800 (CN)
(72) Inventor: He, Xiongwei, Zhuji, Zhejiang Province 311800 (CN)
(74) Representative: Loo, Chi Ching

(56) References cited:
- WO-A1-2016/126607
- WO-A1-2016/126607
- CN-A- 107 550 535
- US-A1- 2015 057 704
- US-B2- 8 062 311

## Description

### Field of the Invention

The invention relates to the field of minimally invasive surgical instruments in the medical field, in particular to a large wound area anastomosis clip.

### Description of the Related Art

In minimally invasive surgery, the wound requires a hemostasis operation to be performed thereon. At this time, the anastomosis clip with a clip is generally used to clamp the wound to close it to achieve hemostasis. However, the existing anastomosis clip has strict requirements on the size of the wound. When the wound surface is too large, the ordinary anastomosis clip cannot clamp the tissue at both ends of the wound and clamp and polymerize due to the limitation of the opening width of the clip. Therefore, for the hemostasis of the large wound surface in minimally invasive surgery, the fast and effective hemostasis method of the anastomosis clip cannot be adopted in the prior art. Therefore, there is an urgent need for an anastomosis clip suitable for hemostasis of the large wound surface. US 2015/057704 discloses a closure device. WO2016/126607 discloses a triple pronged clip.

### BRIEF SUMMARY OF THE INVENTION

The invention is defined in independent claim 1. Certain optional features of the invention are defined in the dependent claims. The object of the present disclosure is to provide a large wound area anastomosis clip which can be applied to the clamping hemostasis of a large wound and which also has all the advantages of the clamp type anastomosis clip.

The technical solution adopted to solve the above technical problems is: A large wound area anastomosis clip, comprising:
a shell;
a first slip ring and a second slip ring slidably coupled to the shell;
a first sleeve fixed on one end of the shell;
a second sleeve connected to the first sleeve;
a clip having a first clip portion and a second clip portion, the first clip portion is connected to the first slip ring by a first connecting member, the second clip portion is connected to the second slip ring by a second connecting member, the first connecting member and the second connecting member pass through the first sleeve and the second sleeve simultaneously;
an intermediate post which partially protrudes outside the second sleeve, and the intermediate post is located between the first clip portion and the second clip portion;
   the first slip ring can pull the first clip portion into the second sleeve, the first clip portion and the intermediate post form a first clamping zone, the second slip ring can pull the second clip portion into the second sleeve, the second clip portion and the intermediate post form a second clamping zone, and the first clip portion and the second clip portion are retracted into a certain position and are respectively disengaged from the first connecting member and the second connecting member, characterised in that the intermediate post is fixed on the second sleeve; the second sleeve is releasably connected to the first sleeve by a trip mechanism that is releasable, the trip mechanism comprising a partition plate and a hook rod, the partition plate being fixed inside the first sleeve, the hook rod having a bent middle portion which is spanned and fixed to the partition plate and two curved-shaped ends, wherein each the first sleeve and the second sleeve have two connecting holes, the two ends of the hook rod inserted in-use into the corresponding connecting holes, thereby realizing a connection between the first sleeve and the second sleeve, the partition plate having a first and second through holes through which the first connecting member and the second connecting member can pass respectively, each of the first and second through holes having an inner diameter, each of the first connecting member and the second connecting member having: a first portion which has a width smaller than the inner diameter so as to be passable through the corresponding through hole, and a second portion which has a width greater than the inner diameter, the second portion being positioned between the trip mechanism and the clip; the second portion of each of the first connecting member and the second connecting member being able to bear against the partition plate of the trip mechanism so that continued pulling of at least one of the first slip ring and the second slip ring allows the trip mechanism to disengage from the second sleeve.

In a preferred embodiment, the first portion of the first connecting member may include a first pull rod, and the second portion of the first connecting member may comprise a first steel wire and a first boosting tube, the first pull rod, the first steel wire and the first boosting tube may be connected in series, one end of the first pull rod may be connected to the first clip portion, the first boosting tube may be fixedly connected with the first slip ring; the first portion of the second connecting member may include a second pull rod, and the second portion of the second connecting member comprising a second steel wire and a second boosting tube, the second pull rod, the second steel wire and the second boosting tube may be connected in series, one end of the second pull rod may be connected to the second clip portion, and the second boosting tube may be fixedly connected with the second slip ring.

In a preferred embodiment, one end of the first pull rod and one end of the second pull rod both have a hook-shaped piece which can be deformed and straightened after being subjected to a certain pulling force, a rivet may be fixedly disposed at one end of the first clip portion and one end of the second clip portion, the hook-shaped piece on the first pull rod hooks the rivet on the first clip portion, the hook-shaped piece on the second pull rod hooks the rivet on the second clip portion, one end of the second sleeve has two locating holes, and the rivets can be inserted into locating holes to locate the first clip portion and the second clip portion.

In a preferred embodiment, the first sleeve may include a transition sleeve, a reducer sleeve and a flexible tube, the transition sleeve may be connected to the second sleeve by the trip mechanism, the transition sleeve may be connected to the flexible tube by the reducer sleeve, the flexible tube may be connected to the shell, and the flexible tube has two mutually separated passages for separating the first connecting member and the second connecting member.

In a preferred embodiment, the shell may include a conical barrel, a first core rod, a second core rod and a hand ring, the first core rod and the second core rod may be relatively mounted, the first slip ring and the second slip ring may be slidably disposed between the first core rod and the second core rod, and the conical barrel and the hand ring may be respectively fixed to two ends of a structure formed by a fastening of the first core rod and the second core rod.

In a preferred embodiment, the first clip portion and the second clip portion may be both in a same structure and shape and each have an arcuate structure; by pulling the first clip portion and the second clip portion into the second sleeve, the restricting action of a sidewall of the second sleeve can gradually straighten the curved structure, so that the first clip portion and the second clip portion may be respectively engaged with the intermediate post.

The invention has the beneficial effects that the first slip ring can pull the first clip into the second sleeve, and the first clip and the middle column form a first clamping zone, and the second slip ring can be pulled The second clip portion is retracted into the second sleeve, and the second clip portion and the intermediate post form a second clamping zone, and the first clip portion and the second clip portion are retracted into a certain position and respectively connected to the first connecting member and the second connecting member The connecting member is disengaged and continues to pull at least one of the first slip ring and the second slip ring to disengage the connection mechanism from the second sleeve. In operation, the first clamping zone formed by the first clip portion and the intermediate post is used to clamp the tissue at one end of the wound, and the end tissue is moved to the side of the opposite end tissue, which is formed by using the second clip portion and the middle column. The second clamping zone clamps the opposite end tissue to achieve hemostasis of the large wound surface. At the same time, the anastomotic clip of the present invention also has all the advantages of a conventional anastomotic clip.

### BRIEF DESCRIPTION OF THE DRAWINGS

Figure 1 is a perspective view of the anastomotic clip according to embodiment 1, not in accordance with the present invention;
Figure 2 is a perspective view of the second sleeve in the first embodiment not in accordance with the present invention;
Figure 3 is a partial enlarged view of the first pull rod and the second pull rod of Figure 2;
Figure 4 is a cross-sectional view showing the trip sleeve of the first embodiment not in accordance with the present invention;
Figure 5 is a partial enlarged view of the flange and the snap ring of Figure 4;
Figure 6 is a perspective view of the shell of the first embodiment not in accordance with the present invention;
Figure 7 is a cross-sectional view showing the shell of the first embodiment not in accordance with the present invention;
Figure 8 is a perspective view of the lower portion of the flexible tube in the second embodiment in accordance with the present invention;
Figure 9 is a perspective view of the connection/trip mechanism of the second embodiment in accordance with the present invention;
Figure 10 is a perspective view of the connection/trip mechanism of embodiment 2 in accordance with the present invention.

The reference numerals in the figure are: 1: shell, 11: conical barrel, 12: core rod, 121: first core rod, 1211: first passage, 13: hand ring, 21: first slip ring, 22: second slip ring, 31: first sleeve, 311: transition sleeve, 312: reducer sleeve, 313: flexible tube, 314: connecting sleeve, 315: connecting tube, 32: second sleeve, 321: locating hole, 322: snap ring, 323: connecting hole, 41: first clip portion, 42: second clip portion, 5: intermediate post, 51: pin, 6: positioning mechanism, 61: tightening nut, 62: fixed length block, 63: linkage shaft, 631: step surface, 711, 721: rivet, 712: first pull rod, 7121, 7221: hook-shaped piece, 713: first steel wire, 714: first boosting tube, 722: second pull rod, 723: second steel wire, 724: second boosting tube, 81: trip sleeve, 811: flange, 812: first through hole, 821: partition plate, 8211: second through hole, 822: hook rod.

### DETAILED DESCRIPTION OF THE INVENTION

The invention will now be described in detail in conjunction with the drawings.

### Example 1

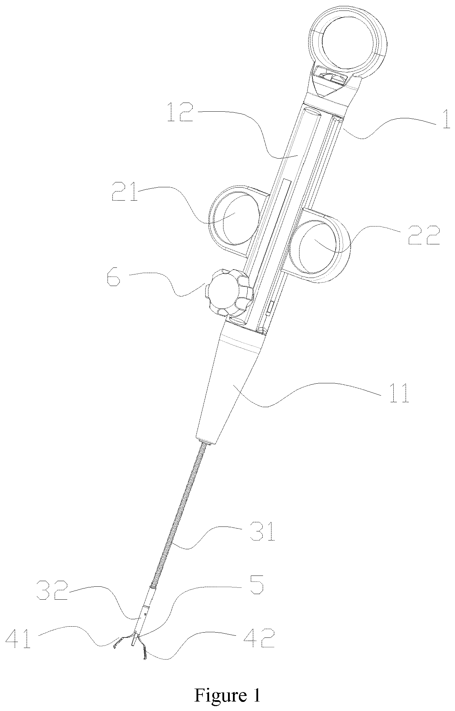

As shown in FIG. 1 , a perspective view of the anastomotic clip according to an embodiment not in accordance with the present invention, the anastomotic clip includes a housing or shell 1, a first slip ring 21 , a second slip ring 22 , a first sleeve 31 , and a second sleeve 32 . The first clip portion 41, the second clip portion 42 and the intermediate post 5, preferably, a positioning mechanism 6 is also mounted on the shell 1. The first slip ring 21 and the second slip ring 22 are slidably coupled to the shell 1. A first sleeve 31 is fixed to one end of the shell 1, and a second sleeve 32 is connected to the first sleeve 31 by a trip/connection mechanism. At the end of the second sleeve 32, there are a first clip portion 41 and a second clip portion 42 having opposite bending directions, and the first clip portion 41 is connected to the first slip ring by a first connecting member (not shown). The second clip portion 42 is coupled to the second slip ring 22 by a second connector (not shown). An intermediate post 5 is mounted between the first clip portion 41 and the second clip portion 42 and is fixed in the second sleeve 32 and partially exposed outside the second sleeve 32.

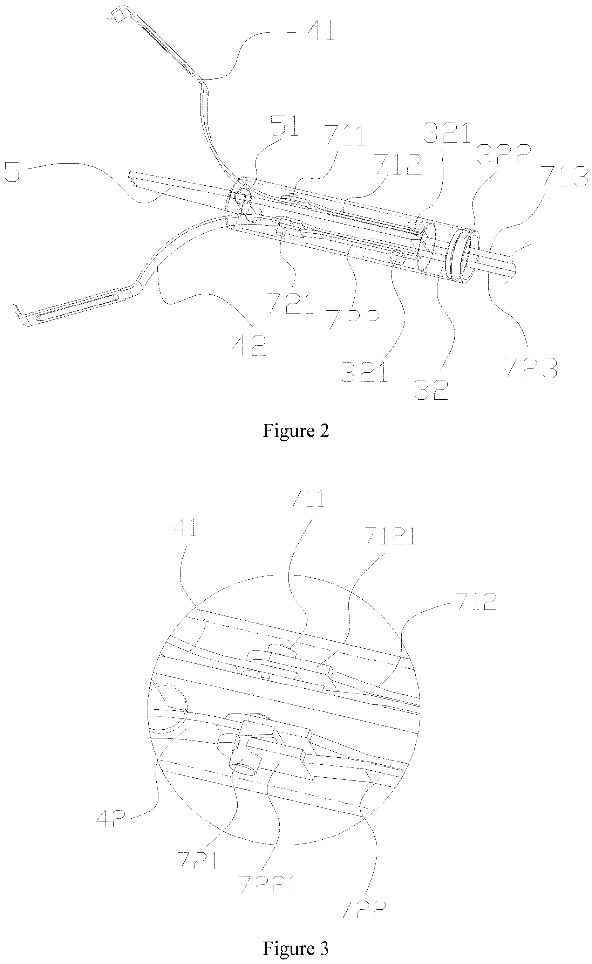

The principle of clamping and positioning structure of the anastomotic clip will be specifically described with reference to FIG. 2 and FIG. 3. The intermediate column 5 is fixed to the inside of the second sleeve 32 by a pin 51. The first clip portion 41 and the second clip portion 42 are both arc-shaped, and the bending directions of the two clips are opposite. The rivets 711 and 721 are fixed at the ends of the first clip portion 41 and the second clip portion 42 and are connected to the rivet 711. There is a first pull rod 712, a second pull rod 722 is connected to the rivet 721, and a first steel wire 713 is fixedly connected to the end of the first pull rod 712, and a second steel wire 723 is fixedly connected to the end of the second pull rod 722. As shown in FIG. 3, the first pull rod 712 and the second pull rod 722 are connected to the rivet, and each has a hook-shaped piece 7121, 7221, and the hook-shaped piece can be deformed and straightened after being subjected to a certain pulling force. Therefore, when the first steel wire 713 pulls the first pull rod 712 to contract inwardly, the first clip portion 41 can be pulled by the rivet 711 to contract the inside of the second sleeve 32, due to the limit of the side wall of the second sleeve 32 and The elastic deformation of the first clip portion 41, the head of the first clip portion 41 approaches the intermediate post 5 to form a clamping action; since the first clip portion 41 is retracted into the second sleeve 32, due to its existence elastically, the portion of the first clip portion 41 that is coupled to the rivet 711 will move away from the intermediate axis. Gradually, the rivet 711 will bear against the inner wall of the second sleeve 32 and exert pressure on the inner wall thereof. When the rivet 711 is moved to the position of the locating hole 321 opened at the end of the second sleeve, the rivet 711 is pressed into the locating hole 321 due to the elastic force of the first clip portion 41, and the first steel wire 713 is continuously pulled, since the rivet 711 has been stuck. If the first clip portion 41 is unable to be further retracted, the first pull rod 712 is deformed by the force of the hook-shaped piece 7121, so as to be disengaged from the rivet 711, thereby disengaging the first connecting member. The first clip portion 41 is stuck and cannot be re-launched. Since the structure and connection relationship of the second clip portion 42 and the second connector connected thereto are the same as those of the first clip portion 41, the clamping and disengaging process is also the same as that of the first clip portion 41, and therefore will not be described again.

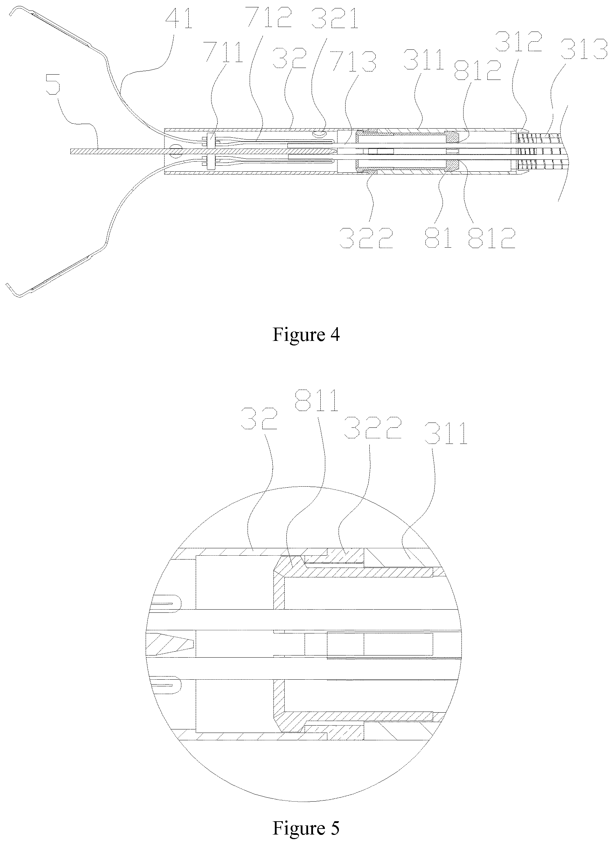

The structure and principle of the first sleeve 31 and the second sleeve 32 of the anastomotic clip are specifically described in conjunction with FIGS. 4 and 5. The first sleeve 31 includes a transition sleeve 311, a reducer sleeve 312 and a flexible tube 313 which are sequentially connected. Preferably, the flexible tube 313 is a spring tube, and the flexible tube 313 is fixed on the shell 1, and the transition sleeve 311 is coupled to the second sleeve 32 by a trip sleeve 81. A snap ring 322 is fixed to one end of the second sleeve 32. The snap ring 322 has an engaging surface having an inner diameter smaller than the inner diameter of the second sleeve 32, and corresponding to the engaging surface, the trip sleeve 81 is disposed on the trip sleeve 81. The flange 811 is fastened and one end of the trip sleeve 81 is fixed inside the transition sleeve 311. The first sleeve 31 and the second sleeve 32 are connected by the cooperation of the snap ring 322 and the trip sleeve 81. And a first through hole 812 is formed at one end of the trip sleeve 81. The first through hole 812 has two, and the first steel wire 713 and the second wire 723 are passed through the first through hole 812, and the first through hole is ensured. The diameter of the 812 should be less than the width of the first pull rod 712 and the second pull rod 722. After both the first pull rod 712 and the second pull rod 722 are disengaged from the rivet, under the pulling of the first steel wire 713 and the second wire 723, the first pull rod 712 and the second pull rod 722 continue to pull inwardly, when the first pull rod 712 and When the end surface of the second pull rod 722 bears against the first through hole 812 of the trip sleeve 81, the first pull rod 712 and the second pull rod 722 can pull the strip sleeve inwardly due to the limitation of the first through hole 812. Since the flange 811 has the property of being deformed by force, it is disengaged from the engaging surface of the snap ring 322, so that the connection between the transition sleeve 311 and the second sleeve 32 is broken, and the trip is realized.

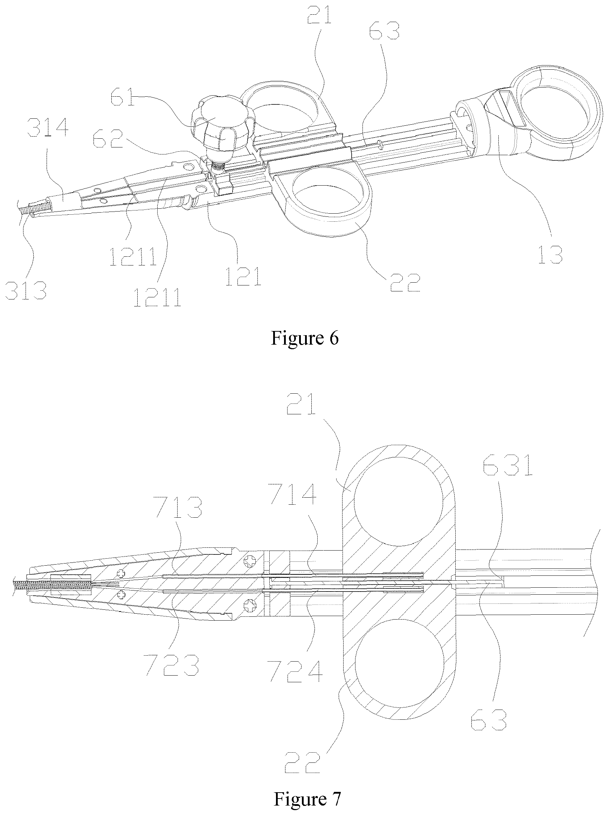

The structure and operation principle of clamping and tripping at the shell 1 using the first slip ring 21 and the second slip ring 22 will be specifically described with reference to FIGS. 6 and 7. The shell 1 includes a conical cylinder 11, a core rod 12, and a hand ring 13, and the conical cylinder 11, the core rod 12, and the hand ring 13 are sequentially connected. The core rod 12 includes a first core rod 121 and a second core rod, and the first core rod 121 and the second core rod each have a rail for the first slip ring 21, the second slip ring 22, and the ruler 62 slides. The flexible tube 313 is fixed in the core rod 12 by the connecting sleeve 314, and the spacing required for the first steel wire 713 and the second wire 723 to connect the first slip ring 21 and the second slip ring 22 in the core rod 12 is expanded. A passage is formed, and the passage is formed by combining the first passage 1211 and the second passage respectively formed on the first core rod 121 and the second core rod. The first steel wire 713 and the first slip ring 21 are connected by a first boosting tube 714, and the second wire 723 and the second slip ring 22 are connected by a second boosting tube 724. Since the first slip ring 21 and the second slip ring 22 are combined to have a circular passage, the linkage shaft 63 can pass between the first slip ring 21 and the second slip ring 22 and fix the fixed length block 62 at one end. A fixed nut 61 is mounted on the fixed block 62, and the tightening nut 61 can rotate and abut against the surface of the second core rod, and has friction with the surface of the second core rod due to pressure, thereby limiting the fixed length block 62 sliding on the track. At the other end of the linkage shaft 63, there is a step surface 631, and the width of the step surface 631 is larger than the diameter of the first slip ring 21 and the second slip ring 22 to form a channel, so that it can withstand the first slip ring 21 and The second slip ring 22 prevents it from sliding further back.

Hereinafter, the method of using the anastomosis clip will be specifically described. First, in the preparation for hemostasis surgery, it is necessary to tighten and tighten the nut 61 to position the fixed length block 62, and then push the first slip ring 21 and the second slip ring 22 so that the first clip portion 41 and the second clip portion 42 are both at Open state. Thereafter, the anastomosis clip is placed on the surface of the tissue through the clamp, and the first clip portion 41 and the intermediate post 5 are pushed to one side of the wound, and the first slip ring 21 is pulled to clamp the first clip portion 41 toward the intermediate post 5 Thereby, the first clip portion 41 and the intermediate post 5 are clamped to the tissue on the side of the wound. At this time, the rivet 711 is caught in the locating hole 321, and the first pull rod 712 is pulled off from the rivet 711, and the first clip portion 41 is stuck. The entire anastomosis clip is moved to the other side of the wound, and the second clip portion 42 and the intermediate post 5 are located on both sides of the edge of the other side of the wound, and the second slip ring 22 is pulled to perform the same action as the first slip ring 21 on the tissue. Clamping. After the first pull rod 712 and the second pull rod 722 are completely pulled off, the nut 61 is loosened and tightened, so that the fixed block 62 can slide freely along the track. At this time, the first slip ring 21 and the second slip ring 22 are simultaneously pulled. The top ends of the first pull rod 712 and the second pull rod 722 will bear against the top surface of the strip sleeve and deform the flange 811 to escape from the snap ring 322. Thereafter, the first sleeve 31 and the second sleeve 32 will be separated, and the first clip portion 41, the second clip portion 42 and the intermediate post 5 clamp the wound to stop bleeding, and the locating hole 321 and the rivet on the second sleeve 32 are engaged. It is also left in the body for fixing the first clip portion 41 and the second clip portion 42, and the rest is pulled out of the body through the forceps to complete the hemostasis operation of the large wound surface.

### Example 2

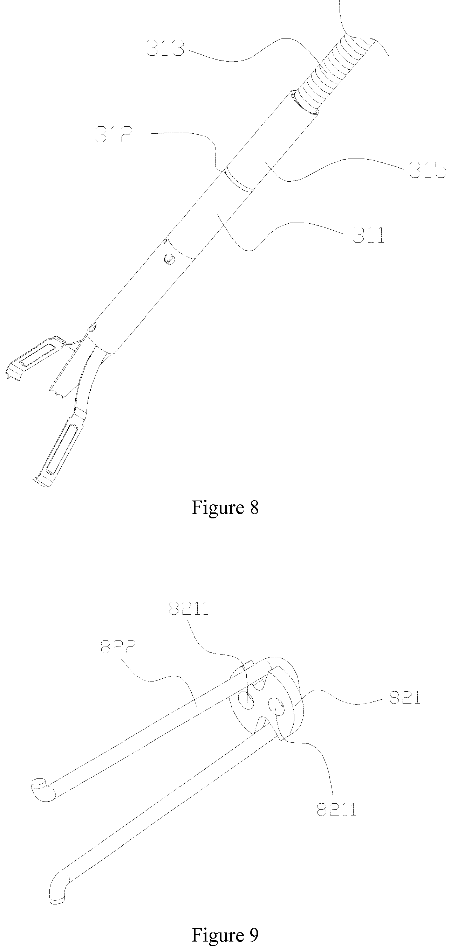

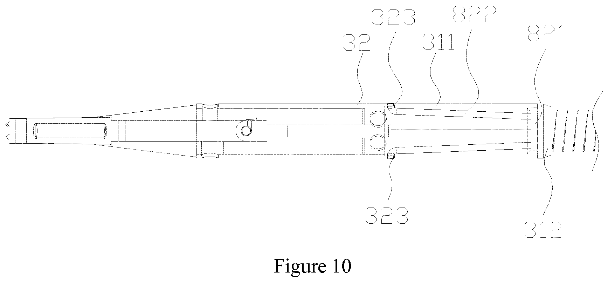

This embodiment is basically the same as Embodiment 1, and only differences from Embodiment 1 will be described below. As shown in FIG. 8, a connecting tube 315 is further mounted on the reducing sleeve 312, and the connecting tube 315 is sleeved outside a part of the flexible tube 313. As shown in FIGS. 9 and 10, the trip mechanism includes a partition plate 821 and a hook rod 822 which is bent in the middle to form a "sever" shape and passes through a groove provided on the partition plate 821. The middle portion of the hook rod 822 is spanned and fixed to the partition plate 821, and has a curved shape at both ends of the hook rod 822. At the joint portion of the second sleeve 32 and the transition sleeve 311, there are connecting holes 323 communicating with each other, and the connecting plate is fixed to the reducer sleeve 312, and both ends of the hook rod 822 are hooked into the connecting hole 323. The first sleeve 31 and the second sleeve 32 are connected. The partition plate has a first through hole 812 and a second through hole 8211 corresponding to the positions of the first pull rod 712 and the second pull rod 722, and the diameters of the first through hole 812 and the second through hole 8211 should be smaller than the first pull rod 712 And the width of the second pull rod 722.

After the first pull rod 712 and the second pull rod 722 are pulled off, further pulling, the first pull rod 712 and the second pull rod 722 can bear against the partition plate 821. The curved shape of both ends of the hook rod 822 is straightened due to the deformation of the external force, so that the second sleeve 32 and the transition tube lose the connection force to achieve the trip.

The above description is only a preferred embodiment of the present invention, and thus does not limit the scope of patent protection of the present invention. Any equivalent structural transformation made by using the specification and the drawings of the present invention is directly or indirectly applied to other related technical fields. The same is included in the scope of protection of the present invention.

## Claims

1. A large wound area anastomosis clip, comprising:
a shell (1);
a first slip ring (21) and a second slip ring (22) slidably coupled to the shell (1);
a first sleeve (31) fixed on one end of the shell (1);
a second sleeve (32) connected to the first sleeve (31);
a clip having a first clip portion (41) and a second clip portion (42), the first clip portion (41) is connected to the first slip ring (21) by a first connecting member, the second clip portion (42) is connected to the second slip ring (22) by a second connecting member, the first connecting member and the second connecting member pass through the first sleeve and the second sleeve simultaneously;
an intermediate post (5) which partially protrudes outside the second sleeve (32), and the intermediate post (5) is located between the first clip portion (41) and the second clip portion (42);
the first slip ring (21) can pull the first clip portion (41) into the second sleeve (32), the first clip portion (41) and the intermediate post (5) form a first clamping zone, the second slip ring (22) can pull the second clip portion (42) into the second sleeve (32), the second clip portion (42) and the intermediate post (5) form a second clamping zone, and the first clip portion (41) and the second clip portion (42) are retracted into a certain position and are respectively disengaged from the first connecting member and the second connecting member, **characterised in that**
the intermediate post (5) is fixed on the second sleeve (32);
the second sleeve (32) is releasably connected to the first sleeve (31) by a trip mechanism that is releasable, the trip mechanism comprising
a partition plate (821) and a hook rod (822), the partition plate (821) being fixed inside the first sleeve (31), the hook rod (822) having a bent middle portion which is spanned and fixed to the partition plate (821) and two curved-shaped ends,
wherein each of the first sleeve (31) and the second sleeve (32) have two connecting holes (323), the two ends of the hook rod (822) inserted in-use into the corresponding connecting holes (323), thereby realizing a connection between the first sleeve (31) and the second sleeve (32), the partition plate (821) having a first and second through holes (8211) through which the first connecting member and the second connecting member can pass respectively, each of the first and second through holes (8211) having an inner diameter,
each of the first connecting member and the second connecting member having:
a first portion which has a width smaller than the inner diameter so as to be passable through the corresponding through hole (8211), and
a second portion which has a width greater than the inner diameter, the second portion being positioned between the trip mechanism and the clip; the second portion of each of the first connecting member and the second connecting member being able to bear against the partition plate (821) of the trip mechanism so that continued pulling of at least one of the first slip ring (21) and the second slip ring (22) allows the trip mechanism to disengage from the second sleeve (32).

2. A large wound area anastomosis clip according to claim 1 wherein the first portion of the first connecting member comprises a first pull rod (712), and the second portion of the first connecting member comprises a first steel wire (713) and a first boosting tube, the first pull rod (712), the first steel wire (713) and the first boosting tube (714) are connected in series, one end of the first pull rod (713) is connected to the first clip portion (41), the first boosting tube (714) is fixedly connected with the first slip ring (21); the first portion of the second connecting member comprises a second pull rod (722), and the second portion of the second connecting member comprising a second steel wire (723) and a second boosting tube, the second pull rod (722), the second steel wire (723) and the second boosting tube (724) are connected in series, one end of the second pull rod (722) is connected to the second clip portion (42), and the second boosting tube (724) is fixedly connected with the second slip ring (22).

3. A large wound area anastomosis clip according to claim 2, wherein one end of the first pull rod (712) and one end of the second pull rod (722) both have a hook-shaped piece (7121, 7221) which can be deformed and straightened after being subjected to a certain pulling force, a rivet (711, 721) is fixedly disposed at one end of the first clip portion (41) and one end of the second clip portion (42), the hook-shaped piece (7121) on the first pull rod (712) hooks the rivet (711) on the first clip portion (41), the hook-shaped piece (7221) on the second pull rod (722) hooks the rivet (721) on the second clip portion (42), one end of the second sleeve (32) has two locating holes (321), and the rivets (711, 712) can be inserted into locating holes (321) to locate the first clip portion (41) and the second clip portion (42).

4. A large wound area anastomosis clip according to claim 3, wherein the first sleeve (31) comprises a transition sleeve (311), a reducer sleeve (312) and a flexible tube (313), the transition sleeve (311) is connected to the second sleeve (32) by the trip mechanism, the transition sleeve (311) is connected to the flexible tube (313) by the reducer sleeve (312), the flexible tube (313) is connected to the shell (1), and the flexible tube (313) has two mutually separated passages for separating the first connecting member and the second connecting member.

5. A large wound area anastomosis clip according to claim 1 wherein the shell (1) comprises a conical barrel (11), a first core rod (121), a second core rod and a hand ring (13), the first core rod (121) and the second core rod are relatively mounted, the first slip ring (21) and the second slip ring (22) are slidably disposed between the first core rod (121) and the second core rod, and the conical barrel (11) and the hand ring (13) are respectively fixed to two ends of a structure formed by a fastening of the first core rod (121) and the second core rod.

6. A large wound area anastomosis clip according to any of claims 1 to 5, wherein the first clip portion (41) and the second clip portion (42) are both in a same structure and shape and each have an arcuate structure; by pulling the first clip portion (41) and the second clip portion (42) into the second sleeve (32), the restricting action of a sidewall of the second sleeve (32) can gradually straighten the curved structure, so that the first clip portion (41) and the second clip portion (42) are respectively engaged with the intermediate post (5).

## Patentansprüche

1. Anastomoseclip für eine große Wundfläche, der Folgendes umfasst:
eine Hülle (1);
einen ersten Schleifring (21) und einen zweiten Schleifring (22), die verschiebbar mit der Hülle (1) gekoppelt sind;
eine erste Hülse (31), die an einem Ende der Hülle (1) angebracht ist;
eine zweite Hülse (32), die mit der ersten Hülse (31) verbunden ist;
einen Clip, die einen ersten Clipabschnitt (41) und einen zweiten Clipabschnitt (42) aufweist, wobei der erste Clipabschnitt (41) mit dem ersten Schleifring (21) durch ein erstes Verbindungselement verbunden ist, der zweite Clipabschnitt (42) mit dem zweiten Schleifring (22) durch ein zweites Verbindungselement verbunden ist, das erste Verbindungselement und das zweite Verbindungselement gleichzeitig durch die erste Hülse und die zweite Hülse hindurchgehen;
einen Zwischenstift (5), der aus der zweiten Hülse (32) teilweise herausragt, und der Zwischenstift (5) sich zwischen dem ersten Clipabschnitt (41) und dem zweiten Clipabschnitt (42) befindet;
wobei der erste Schleifring (21) den ersten Clipabschnitt (41) in die zweite Hülse (32) ziehen kann, der erste Clipabschnitt (41) und der Zwischenstift (5) eine erste Klemmzone ausbilden, der zweite Schleifring (22) den zweiten Clipabschnitt (42) in die zweite Hülse (32) ziehen kann, der zweite Clipabschnitt (42) und der Zwischenstift (5) eine zweite Klemmzone ausbilden und der erste Clipabschnitt (41) und der zweite Clipabschnitt (42) in eine bestimmte Position eingefahren sind und sich jeweils von dem ersten Verbindungselement und dem zweiten Verbindungselement lösen, **dadurch gekennzeichnet, dass dadurch gekennzeichnet, dass**
der Zwischenstift (5) an der zweiten Hülse (32) angebracht ist;
die zweite Hülse (32) mit der ersten Hülse (31) durch einen Auslösemechanismus der lösbar ist, lösbar verbunden ist, wobei der Auslösemechanismus Folgendes umfasst:
eine Trennplatte (821) und eine Hakenstange (822), wobei die Trennplatte (821) innerhalb der ersten Hülse (31) angebracht ist, die Hakenstange (822) einen gebogenen Mittelabschnitt, der gespannt und an der Trennplatte angebracht ist (821), und zwei kurvenförmige Enden aufweist,
wobei jede der ersten Hülse (31) und der zweiten Hülse (32) zwei Verbindungslöcher (323) aufweist, die zwei Enden der Hakenstange (822) in Verwendung in die entsprechenden Verbindungslöcher (323) eingeführt werden, wobei dadurch eine Verbindung zwischen der ersten Hülse (31) und der zweiten Hülse (32) realisiert wird, die Trennplatte (821) ein erstes und ein zweites Durchgangsloch (8211) aufweist, durch die das erste Verbindungselement beziehungsweise das zweite Verbindungselement hindurchgehen kann, wobei jedes des ersten und des zweiten Durchgangslochs (8211) einen Innendurchmesser aufweist,
wobei jedes des ersten Verbindungselements und des zweiten Verbindungselements Folgendes aufweist:
einen ersten Abschnitt, der eine Breite aufweist, die kleiner als der Innendurchmesser ist, sodass er durch das entsprechende Durchgangsloch (8211) hindurchgehen kann und
einen zweiten Abschnitt, der eine Breite aufweist, die über dem Innendurchmesser liegt, wobei der zweite Abschnitt zwischen dem Auslösemechanismus und dem Clip positioniert ist; der zweite Abschnitt jedes des ersten Verbindungselements und des zweiten Verbindungselements in der Lage ist, gegen die Trennplatte (821) des Auslösemechanismus zu drücken, sodass das kontinuierliche Ziehen des ersten Schleifrings (21) und/oder des zweiten Schleifrings (22) dem Auslösemechanismus ermöglicht, sich von der zweiten Hülse (32) zu lösen.

2. Anastomoseclip für eine große Wundfläche nach Anspruch 1, wobei der erste Abschnitt des ersten Verbindungselements eine erste Zugstange (712) umfasst und der zweite Abschnitt des ersten Verbindungselements einen ersten Stahldraht (713) und ein erstes Verstärkungsrohr umfasst, die erste Zugstange (712), der erste Stahldraht (713) und das erste Verstärkungsrohr (714) in Reihe geschaltet sind, ein Ende der ersten Zugstange (713) mit dem ersten Clipabschnitt (41) verbunden ist, das erste Verstärkungsrohr (714) mit dem ersten Schleifring (21) fest verbunden ist; der erste Abschnitt des zweiten Verbindungselements eine zweite Zugstange (722) umfasst und der zweite Abschnitt des zweiten Verbindungselements einen zweiten Stahldraht (723) und ein zweites Verstärkungsrohr umfasst, die zweite Zugstange (722), der zweite Stahldraht (723) und das zweite Verstärkungsrohr (724) in Reihe geschaltet sind, ein Ende der zweiten Zugstange (722) mit dem zweiten Clipabschnitt (42) verbunden ist und das zweite Verstärkungsrohr (724) mit dem zweiten Schleifring (22) fest verbunden ist.

3. Anastomoseclip für eine große Wundfläche nach Anspruch 2, wobei ein Ende der ersten Zugstange (712) und ein Ende der zweiten Zugstange (722) beide ein hakenförmiges Stück (7121, 7221) aufweisen, das verformt und geradegerichtet werden kann nachdem es einer bestimmten Zugkraft ausgesetzt wird, ein Niet (711, 721) an einem Ende des ersten Clipabschnitts (41) und einem Ende des zweiten Clipabschnitts (42) fest eingerichtet ist, das hakenförmige Stück (7121) an der ersten Zugstange (712) den Niet (711) an dem ersten Clipabschnitt (41) einhakt, das hakenförmige Stück (7221) an der zweiten Zugstange (722) den Niet (721) an dem zweiten Clipabschnitt (42) einhakt, ein Ende der zweiten Hülse (32) zwei Fixierlöcher (321) aufweist, und die Nieten (711, 712) in Fixierlöcher (321) eingeführt werden können, um den ersten Clipabschnitt (41) und den zweiten Clipabschnitt (42) zu fixieren.

4. Anastomoseclip für eine große Wundfläche nach Anspruch 3, wobei die erste Hülse (31) eine Übergangshülse (311), eine Reduzierhülse (312) und einen flexibles Rohr (313) umfasst, wobei die Übergangshülse (311) mit der zweiten Hülse (32) durch den Auslösemechanismus verbunden ist, die Übergangshülse (311) mit dem flexiblen Rohr (313) durch die Reduzierhülse (312) verbunden ist, das flexible Rohr (313) mit der Hülle (1) verbunden ist, und das flexible Rohr (313) zwei voneinander getrennte Durchgänge zum Trennen des ersten Verbindungselements und des zweiten Verbindungselements aufweist.

5. Anastomoseclip für eine große Wundfläche nach Anspruch 1, wobei die Hülle (1) einen kegelförmigen Schaft (11), eine erste Kernstange (121), eine zweite Kernstange und einen Handring (13) umfasst, die erste Kernstange (121) und die zweite Kernstange relativ montiert sind, der erste Schleifring (21) und der zweite Schleifring (22) zwischen der ersten Kernstange (121) und der zweiten Kernstange verschiebbar eingerichtet sind und der kegelförmigen Schaft (11) und der Handring (13) jeweils an zwei Enden einer Struktur angebracht sind, die durch eine Befestigung der ersten Kernstange (121) und der zweiten Kernstange ausgebildet wird.

6. Anastomoseclip für eine große Wundfläche nach einem der Ansprüche 1 bis 5, wobei der erste Clipabschnitt (41) und der zweite Clipabschnitt (42) beide in einer gleichen Struktur und Form sind und jeweils eine bogenförmige Struktur aufweisen; durch das Ziehen des ersten Clipabschnitts (41) und des zweiten Clipabschnitts (42) in die zweite Hülse (32), die begrenzende Wirkung einer Seitenwand der zweiten Hülse (32) die kurvige Struktur allmählich geraderichten kann, sodass der erste Clipabschnitt (41) und der zweite Clipabschnitt (42) jeweils mit dem Zwischenstift (5) in Eingriff stehen.

## Revendications

1. Clip d'anastomose pour plaie de grande taille, comprenant :
une coque (1) ;
une première bague collectrice (21) et une seconde bague collectrice (22) accouplées de manière coulissante à la coque (1) ;
un premier manchon (31) fixé sur une extrémité de la coque (1) ;
un second manchon (32) relié au premier manchon (31) ;
un clip ayant une première partie de clip (41) et une seconde partie de clip (42), la première partie de clip (41) est reliée à la première bague collectrice (21) par un premier élément de liaison, la seconde partie de clip (42) est reliée à la seconde bague collectrice (22) par un second élément de liaison, le premier élément de liaison et le second élément de liaison passent à travers le premier manchon et le second manchon simultanément ;
un montant intermédiaire (5) qui fait partiellement saillie à l'extérieur du second manchon (32), et le montant intermédiaire (5) est situé entre la première partie de clip (41) et la seconde partie de clip (42) ;
la première bague collectrice (21) peut tirer la première partie de clip (41) dans le second manchon (32), la première partie de clip (41) et le montant intermédiaire (5) forment une première zone de serrage, la seconde bague collectrice (22) peut tirer la seconde partie de clip (42) dans le second manchon (32), la seconde partie de clip (42) et le montant intermédiaire (5) forment une seconde zone de serrage, et la première partie de clip (41) et la seconde partie de clip (42) sont rétractées dans une certaine position et sont respectivement libérées du premier élément de liaison et du second élément de liaison, **caractérisé en ce que**
le montant intermédiaire (5) est fixé sur le second manchon (32) ;
le second manchon (32) est relié de manière détachable au premier manchon (31) par un mécanisme de déclenchement qui est détachable, le mécanisme de déclenchement comprenant
une plaque de séparation (821) et une tige de crochet (822), la plaque de séparation (821) étant fixée à l'intérieur du premier manchon (31), la tige de crochet (822) ayant une partie médiane pliée qui est étendue et fixée à la plaque de séparation (821) et deux extrémités de forme incurvée,
le premier manchon (31) et le second manchon (32) ayant chacun deux trous de liaison (323), les deux extrémités de la tige de crochet (822) étant insérées pendant l'utilisation dans les trous de liaison (323) correspondants, réalisant ainsi une liaison entre le premier manchon (31) et le second manchon (32), la plaque de séparation (821) ayant un premier et un second trous traversants (8211) à travers lesquels le premier élément de liaison et le second élément de liaison peuvent passer respectivement, le premier et le second trous traversants (8211) ayant chacun un diamètre intérieur,
le premier élément de liaison et le second élément de liaison ayant chacun :
une première partie qui a une largeur inférieure au diamètre intérieur de manière à pouvoir être passée à travers le trou traversant (8211) correspondant, et
une seconde partie qui a une largeur supérieure au diamètre intérieur, la seconde partie étant positionnée entre le mécanisme de déclenchement et le clip ; la seconde partie du premier élément de liaison et la seconde partie du second élément de liaison pouvant s'appuyer contre la plaque de séparation (821) du mécanisme de déclenchement de sorte que la traction continue de la première bague collectrice (21) et/ou de la seconde bague collectrice (22) permet au mécanisme de déclenchement d'être libéré du second manchon (32).

2. Clip d'anastomose pour plaie de grande taille selon la revendication 1, dans lequel la première partie du premier élément de liaison comprend une première tige de traction (712), et la seconde partie du premier élément de liaison comprend un premier fil d'acier (713) et un premier tube de surpression, la première tige de traction (712), le premier fil d'acier (713) et le premier tube de surpression (714) sont reliés en série, une extrémité de la première tige de traction (713) est reliée à la première partie de clip (41), le premier tube de surpression (714) est relié de manière fixe à la première bague collectrice (21) ; la première partie du second élément de liaison comprend une seconde tige de traction (722), et la seconde partie du second élément de liaison comprenant un second fil d'acier (723) et un second tube de surpression, la seconde tige de traction (722), le second fil d'acier (723) et le second tube de surpression (724) sont reliés en série, une extrémité de la seconde tige de traction (722) est reliée à la seconde partie de clip (42) et le second tube de surpression (724) est relié de manière fixe à la seconde bague collectrice (22).

3. Clip d'anastomose pour plaie de grande taille selon la revendication 2, dans lequel une extrémité de la première tige de traction (712) et une extrémité de la seconde tige de traction (722) ont toutes deux une pièce en forme de crochet (7121, 7221) qui peut être déformée et redressée après avoir été soumise à une certaine force de traction, un rivet (711, 721) est disposé de manière fixe à une extrémité de la première partie de clip (41) et à une extrémité de la seconde partie de clip (42), la pièce en forme de crochet (7121) sur la première tige de traction (712) accroche le rivet (711) à la première partie de clip (41), la pièce en forme de crochet (7221) sur la seconde tige de traction (722) accroche le rivet (721) à la seconde partie de clip (42), une extrémité du second manchon (32) a deux trous de localisation (321), et les rivets (711, 712) peuvent être insérés dans des trous de localisation (321) pour localiser la première partie de clip (41) et la seconde partie de clip (42).

4. Clip d'anastomose pour plaie de grande taille selon la revendication 3, dans lequel le premier manchon (31) comprend un manchon de transition (311), un manchon réducteur (312) et un tube flexible (313), le manchon de transition (311) est relié au second manchon (32) par le mécanisme de déclenchement, le manchon de transition (311) est relié au tube flexible (313) par le manchon réducteur (312), le tube flexible (313) est relié à la coque (1) et le tube flexible (313) a deux passages séparés l'un de l'autre et destinés à séparer le premier élément de liaison et le second élément de liaison.

5. Clip d'anastomose pour plaie de grande taille selon la revendication 1, dans lequel la coque (1) comprend un cylindre conique (11), une première tige de noyau (121), une seconde tige de noyau et une bague pour main (13), la première tige de noyau (121) et la seconde tige de noyau sont montées de manière relative, la première bague collectrice (21) et la seconde bague collectrice (22) sont disposées de manière coulissante entre la première tige de noyau (121) et la seconde tige de noyau, et le cylindre conique (11) et les bagues pour main (13) sont respectivement fixées à deux extrémités d'une structure formée par une fixation de la première tige de noyau (121) et de la seconde tige de noyau.

6. Clip d'anastomose pour plaie de grande taille selon l'une quelconque des revendications 1 à 5, dans lequel la première partie de clip (41) et la seconde partie de clip (42) sont toutes deux dans des mêmes structure et forme et ont chacune une structure arquée ; en tirant la première partie de clip (41) et la seconde partie de clip (42) dans le second manchon (32), l'action de restriction d'une paroi latérale du second manchon (32) peut redresser progressivement la structure incurvée, de sorte que la première partie de clip (41) et la seconde partie de clip (42) sont respectivement mises en prise avec le montant intermédiaire (5).
